# EUROPEAN PATENT APPLICATION

(11) **EP 4 191 599 A1**
(43) Date of publication of application: **07.06.2023**
(21) Application number: 21851572.4
(22) Date of filing: 07.05.2021
(51) Int. Cl.: G16H 20/10, G16H 50/70

(54) **INFORMATION DETERMINATION METHOD AND APPARATUS**

(30) Priority: 28.07.2020 CN 202010735811
(71) Applicant: BEIJING JINGDONG TUOXIAN TECHNOLOGY CO., LTD., Beijing 100176 (CN)
(72) Inventor: ZHAO, Jun, Beijing 100176 (CN); KUANG, Zhexiang, Beijing 100176 (CN); LIU, Xiaolong, Beijing 100176 (CN); HUANG, Sijin, Beijing 100176 (CN)
(74) Representative: Mewburn Ellis LLP
(86) International application number: PCT/CN2021/092019
(87) International publication number: WO 2022/021990

(57) **Abstract**

An information determination method and apparatus. A specific implementation solution is: acquiring prescription review information (101), wherein the prescription review information comprises: prescription information issued by a doctor and review information given by a pharmacist according to the prescription information; performing feature extraction on the prescription information and the review information to generate a feature data set corresponding to the prescription review information (102); and determining the feature data set according to current review rules to obtain review results corresponding to the prescription review information (103), wherein the review rules are used to characterize a correspondence between the feature data set and the review results, and the review rules are updated on the basis of training results of a classification decision model obtained by training. The solution implements a method for determining the prescription review information by using the review rules obtained by learning, and improves the review efficiency and accuracy of a prescription review system.

## Description

### CROSS-REFERENCE TO RELATED APPLICATIONS

The present disclosure claims priority to Chinese Patent Application No. 202010735811.1 titled "METHOD AND APPARATUS FOR JUDGING INFORMATION" filed on July 28, 2020, the full text of which is incorporated herein by reference.

### TECHNICAL FIELD

The present disclosure relates to the technical field of computers, specifically relates to the technical fields of Wise Information Technology of Med and information processing, and more specifically relates to a method and apparatus for judging information.

### BACKGROUND

At present, due to the non-uniform distribution of medical resources, some hospital pharmacists lack knowledge in the professional field, and less perfectly and accurately audit prescriptions issued by hospitals, such that a prescription auditing system is required to assist in completing the auditing. The core of the prescription auditing system lies in the auditing rule. At present, the auditing rule is commonly acquired by two approaches: one is configuration by pharmacists based on experience, and another is extraction by a system from a pharmacological knowledge graph. The manual rule configuration is a labor-consuming work, and tends to cause incorrect configuration by negligence. As a new method, extracting the rule from the pharmacological knowledge graph can reduce the workload of pharmacists, but the acquired rule is a static rule that fails to dynamically learn the prescription auditing rule, and because each hospital has different auditing rules for the same drug, the rule extracted from the general pharmacological knowledge graph cannot meet the diverse prescription auditing requirements in a plurality of hospitals. Therefore, it appears particularly important to learn the auditing rule from a large amount of prescription comment information in each hospital.

### SUMMARY

The present disclosure provides a method and apparatus for judging information, a device, and a storage medium.

In a first aspect, embodiments of the present disclosure provide a method for judging information, comprising: acquiring prescription comment information, wherein the prescription comment information comprises: prescription information issued by a doctor and comment information given by a pharmacist based on the prescription information; performing feature extraction on the prescription information and the comment information, to generate a feature data set corresponding to the prescription comment information; and judging the feature data set based on a current auditing rule to obtain a comment result corresponding to the prescription comment information, wherein the auditing rule is used for characterizing a corresponding relationship between the feature data set and the comment result, and the auditing rule is updated based on a training result of a classification decision model obtained by training.

In some embodiments, the method further comprises: training the classification decision model based on a preset condition; and the classification decision model is obtained by training based on following steps: acquiring a training sample set, wherein training samples in the training sample set comprise: various feature data sets extracted from different hospitals and comment results corresponding to the various feature data sets of different hospitals; and training to obtain the classification decision model using a machine learning method, using the various feature data sets of different hospitals comprised in the training samples in the training sample set as an input of a detection network, and using the comment results corresponding to the various feature data sets of different hospitals as a desired output of the detection network.

In some embodiments, the auditing rule is updated based on a training result of a classification decision model obtained by training, comprising: judging whether the training of the classification decision model is completed; and updating the auditing rule based on an input parameter and an output parameter of the classification decision model, in response to the training of the classification decision model being completed.

In some embodiments, before the performing feature extraction on the prescription information and the comment information, to generate a feature data set corresponding to the prescription comment information, and before the acquiring a training sample set, the method further comprises: cleaning the prescription information and the comment information to generate cleaned prescription comment information, wherein the cleaning is based on data structuring on the prescription information and the comment information.

In some embodiments, the cleaning the prescription information and the comment information to generate cleaned prescription comment information comprises: performing information extraction on the prescription information to obtain personal information corresponding to the prescription information, diagnostic information corresponding to the prescription information, and drug information corresponding to the prescription information; determining a population tag corresponding to the prescription information based on the personal information; standardizing the diagnostic information to obtain a diagnosis name corresponding to the prescription information; and performing standardization and unit normalization on the drug information to obtain drug standard information corresponding to the prescription information; classifying the comment information based on a text classification technology to determine a category to which the comment information belongs; and generating the cleaned prescription comment information based on the population tag, the diagnosis name, the drug standard information, and the category to which the comment information belongs.

In some embodiments, after the performing feature extraction on the prescription information and the comment information, to generate a feature data set corresponding to the prescription comment information, and after the acquiring the training sample set, the method further comprises: encoding the feature data set to obtain a processed feature data set.

In some embodiments, after the performing feature extraction on the prescription information and the comment information, to generate a feature data set corresponding to the prescription comment information, and after the acquiring the training sample set, the method further comprises: screening the feature data set based on a preset pharmacological knowledge, to generate a screened feature data set.

In some embodiments, the method further comprises: optimizing a product structure and/or a product strategy based on a relevance between the comment result and a product.

In some embodiments, the feature data set includes: discrete features with relatively independent features and associated features with association relationships between features.

In a second aspect, embodiments of the present disclosure provide an apparatus for judging information, comprising: an acquiring unit configured to acquire prescription comment information, wherein the prescription comment information comprises: prescription information issued by a doctor and comment information given by a pharmacist based on the prescription information; a feature extracting unit configured to perform feature extraction on the prescription information and the comment information, to generate a feature data set corresponding to the prescription comment information; and a judging unit configured to judge the feature data set based on a current auditing rule to obtain a comment result corresponding to the prescription comment information, wherein the auditing rule is used for characterizing a corresponding relationship between the feature data set and the comment result, and the auditing rule is updated based on a training result of a classification decision model obtained by training.

In some embodiments, the apparatus further comprises: a training unit configured to train the classification decision model based on a preset condition; and the classification decision model in the training unit is obtained by training based on following steps: acquiring a training sample set, wherein training samples in the training sample set comprise: various feature data sets extracted from different hospitals and comment results corresponding to the various feature data sets of different hospitals; and training to obtain the classification decision model using a machine learning method, using the various feature data sets of different hospitals comprised in the training samples in the training sample set as an input of a detection network, and using the comment results corresponding to the various feature data sets of different hospitals as a desired output of the detection network.

In some embodiments, the judging unit comprises: a judging module configured to judge whether the training of the classification decision model is completed; and an updating module configured to update the auditing rule based on an input parameter and an output parameter of the classification decision model, in response to the training of the classification decision model being completed.

In some embodiments, the apparatus further comprises: a cleaning unit configured to clean the prescription information and the comment information to generate cleaned prescription comment information, wherein the cleaning is based on data structuring on the prescription information and the comment information.

In some embodiments, the cleaning unit comprises: an extracting module configured to perform information extraction on the prescription information to obtain personal information corresponding to the prescription information, diagnostic information corresponding to the prescription information, and drug information corresponding to the prescription information; a determining module configured to determine a population tag corresponding to the prescription information based on the personal information; standardizing the diagnostic information to obtain a diagnosis name corresponding to the prescription information; and performing standardization and unit normalization on the drug information to obtain drug standard information corresponding to the prescription information; a classifying module configured to classify the comment information based on a text classification technology to determine a category to which the comment information belongs; and a generating module configured to generate the cleaned prescription comment information based on the population tag, the diagnosis name, the drug standard information, and the category to which the comment information belongs.

In some embodiments, the apparatus further comprises: a processing unit configured to encode the feature data set to obtain a processed feature data set.

In some embodiments, the apparatus further comprises: a screening unit configured to screen the feature data set based on a preset pharmacological knowledge, to generate a screened feature data set.

In some embodiments, the apparatus further comprises: an optimizing unit configured to optimize a product structure and/or a product strategy based on a relevance between the comment result and a product.

In a third aspect, embodiments of the present disclosure provide an electronic device, comprising: one or more processors; and a memory, storing one or more programs, wherein the one or more programs, when executed by the one or more processors, cause the one or more processors to implement the method provided by the first aspect.

In a fourth aspect, embodiments of the present disclosure provide a computer-readable medium, storing a computer program thereon, wherein the program, when executed by a processor, causes the processor to implement the method provided by the first aspect.

The technology according to the present disclosure acquires prescription comment information, performs feature extraction on prescription information and comment information to generate a feature data set corresponding to the prescription comment information, and judges the feature data set based on a current auditing rule to obtain a comment result corresponding to the prescription comment information, where the auditing rule is updated based on a training result of a classification decision model obtained by training, thereby solving the problem of incorrect configuration caused by manual rule configuration, freeing the pharmacists from the complex rule configuration work, further solving the problem of failure to dynamically learn the prescription auditing rule because the acquired rule in the prior art is a static rule, implementing a method for judging prescription comment information using an auditing rule obtained by learning, and improving the auditing efficiency and accuracy of the prescription auditing system.

It should be understood that contents described in the SUMMARY are neither intended to identify key or important features of embodiments of the present disclosure, nor intended to limit the scope of the present disclosure. Other features of the present disclosure will become readily understood with reference to the following description.

### BRIEF DESCRIPTION OF THE DRAWINGS

The accompanying drawings are used for better understanding of the present solution, and do not impose any limitation on the present disclosure.
Fig. 1 is a schematic diagram of a method for judging information according to a first embodiment of the present disclosure;
Fig. 2 is a scenario diagram in which the method for judging information according to an embodiment of the present disclosure may be implemented;
Fig. 3 is a schematic diagram of the method for judging information according to a second embodiment of the present disclosure;
Fig. 4a is an example diagram of cleaned prescription comment information;
Fig. 4b is an example diagram of an auditing rule;
Fig. 5 is a schematic structural diagram of an apparatus for judging information according to an embodiment of the present disclosure; and
Fig. 6 is a block diagram of an electronic device configured to implement the method for judging information of embodiments of the present disclosure.

### DETAILED DESCRIPTION OF EMBODIMENTS

Example embodiments of the present disclosure are described below with reference to the accompanying drawings, including various details of the embodiments of the present disclosure to contribute to understanding, which should be considered merely as examples. Therefore, those of ordinary skills in the art should realize that various alterations and modifications may be made to the embodiments described here without departing from the scope and spirit of the present disclosure. Similarly, for clearness and conciseness, descriptions of well-known functions and structures are omitted in the following description.

It should be noted that the embodiments in the present disclosure and the features in the embodiments may be combined with each other on a non-conflict basis. The present disclosure will be described in detail below with reference to the drawings and in combination with the embodiments.

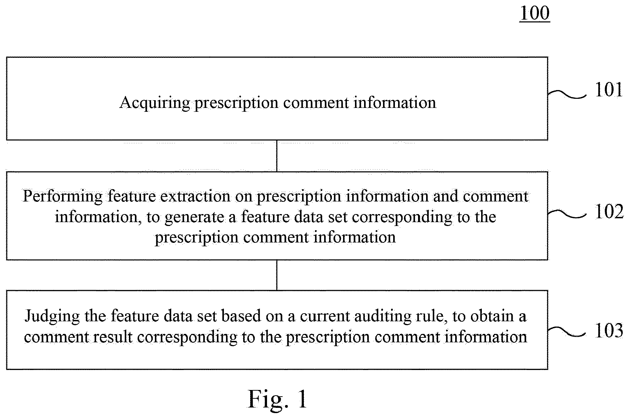

Fig. 1 shows a schematic diagram 100 of a method for judging information according to a first embodiment of the present disclosure. The method for judging information includes the following steps:

Step 101: acquiring prescription comment information.

In the present embodiment, an executing body may acquire the prescription comment information from other electronic devices or locally through a wired connection or a wireless connection, or may obtain the prescription comment information by analyzing a prescription auditing request. The prescription comment information includes: prescription information and comment information. The prescription information is issued by a doctor and includes personal information of a patient (e.g., age and gender), diagnostic information of the patient (including disease, allergic history, and physical examination indicators), and drug information (including generic name, specification, single dose, administration way, and administration frequency of a drug). The comment information is given by a pharmacist based on the prescription information, including judgment information of the pharmacist on whether the prescription is reasonable. The comment information may include: inconsistency between medication and diagnosis, inconsistency between medication and gender, very high single dose, and the like. It should be noted that the wireless connection may include, but is not limited to, 3G, 4G, or 5G connection, WiFi connection, Bluetooth connection, WiMAX connection, Zigbee connection, UWB (ultra wideband) connection, and other wireless connections that are known at present or are to be developed in the future.

Step 102: performing feature extraction on prescription information and comment information, to generate a feature data set corresponding to the prescription comment information.

In the present embodiment, the executing body may perform feature extraction on the prescription information and the comment information based on a feature extracting method, to generate the feature data set corresponding to the prescription comment information. The feature data set includes: discrete features with relatively independent features and associated features with association relationships between features. For example, features in the feature data set may include: gender, age, and population features of the patient; diagnostic feature of the patient; and generic name, administration frequency, single dose, and administration way features of the drug. The gender, population, diagnosis, generic name, and administration way are discrete features, while the age, administration frequency, and single dose are associated features.

Step 103: judging the feature data set based on a current auditing rule, to obtain a comment result corresponding to the prescription comment information.

In the present embodiment, the executing body may judge the feature data set based on the current comment rule, to obtain the comment result corresponding to the prescription comment information. The auditing rule is used for characterizing a corresponding relationship between the feature data set and the comment result, and the auditing rule is updated based on a training result of a classification decision model obtained by training, thereby obtaining an optimized auditing rule by dynamic learning based on the auditing rule.

It should be noted that the above updating method is a well-known technology widely researched and applied at present. The description will not be repeated here.

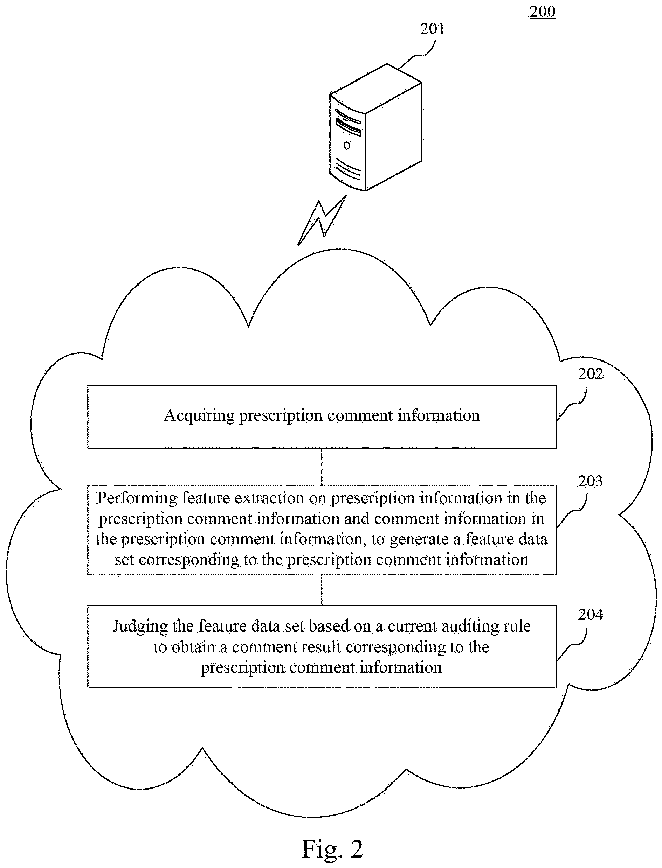

Further referring to Fig. 2, a method 200 for judging information in the present embodiment runs in an electronic device 201. After the electronic device 201 receives an auditing request, the electronic device 201 first acquires prescription comment information 202, then the electronic device 201 performs feature extraction on prescription information in the prescription comment information and comment information in the prescription comment information, to generate a feature data set corresponding to the prescription comment information 203, and finally, the electronic device 201 judges the feature data set based on a current auditing rule to obtain a comment result corresponding to the prescription comment information 204.

The method for judging information provided in the above embodiments of the present disclosure acquires prescription comment information, performs feature extraction on prescription information and comment information to generate a feature data set corresponding to the prescription comment information, and judges the feature data set based on a current auditing rule to obtain a comment result corresponding to the prescription comment information, where the auditing rule is updated based on a training result of a classification decision model obtained by training, thereby solving the problem of incorrect configuration caused by manual rule configuration, freeing the pharmacists from the complex rule configuration work, further solving the problem of failure to dynamically learn the prescription auditing rule because the acquired rule in the prior art is a static rule, implementing a method for judging prescription comment information using an auditing rule obtained by learning, and improving the auditing efficiency and accuracy of the prescription auditing system.

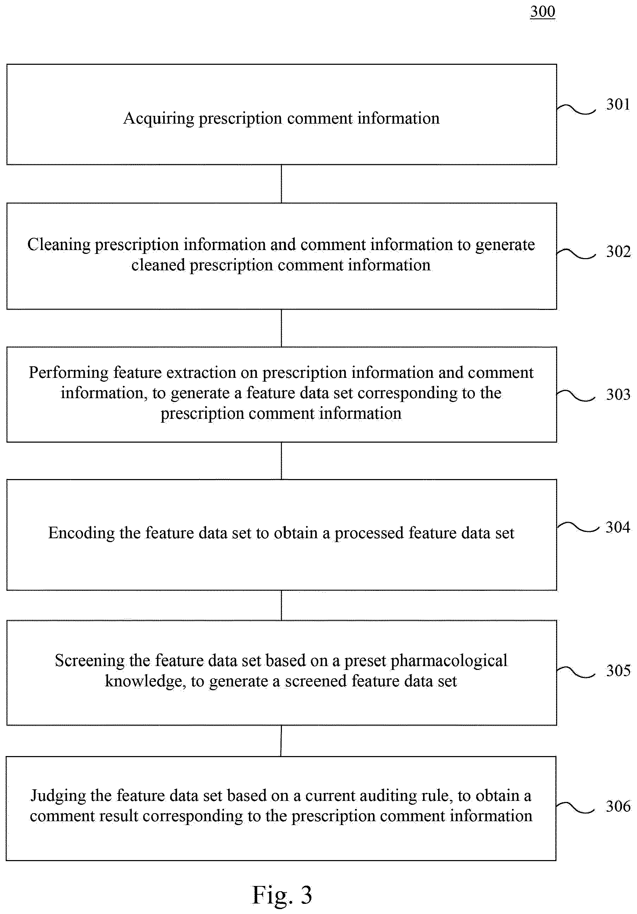

Further referring to Fig. 3, a schematic diagram 300 of the method for judging information according to a second embodiment is shown. The process of the method includes the following steps:
Step 301: acquiring prescription comment information.
Step 302: cleaning prescription information and comment information to generate cleaned prescription comment information.

In the present embodiment, an executing body may clean the prescription information and the comment information respectively, to generate cleaned prescription information and cleaned comment information, and summarize the cleaned prescription information and the cleaned comment information to obtain the cleaned prescription comment information, where the cleaning is based on data structuring on the prescription information and the comment information.

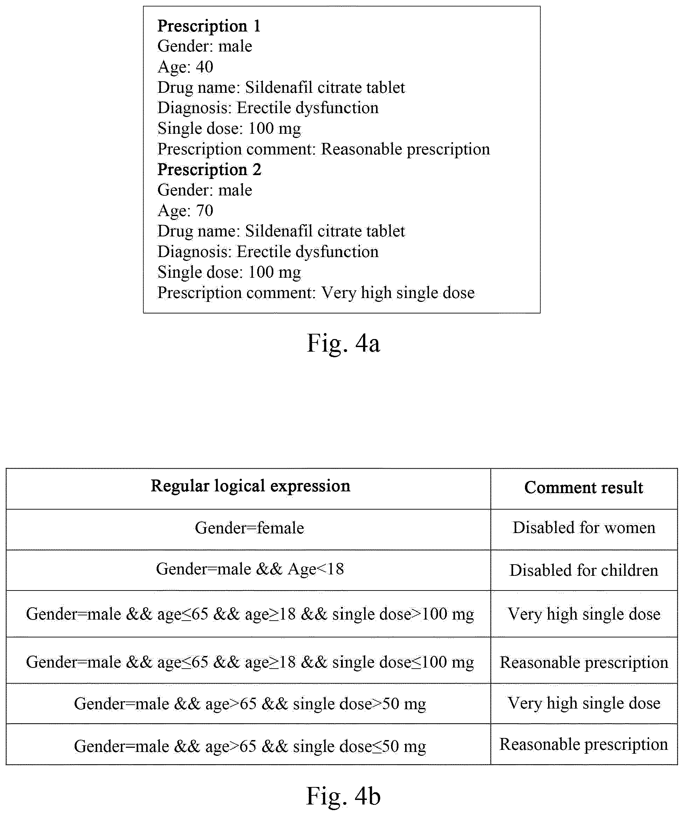

In some alternative implementations of the present embodiment, the cleaning the prescription information and the comment information to generate the cleaned prescription comment information includes: performing information extraction on the prescription information to obtain personal information corresponding to the prescription information, diagnostic information corresponding to the prescription information, and drug information corresponding to the prescription information; determining a population tag corresponding to the prescription information based on the personal information; standardizing the diagnostic information to obtain a diagnosis name corresponding to the prescription information; and performing standardization and unit normalization on the drug information to obtain drug standard information corresponding to the prescription information; classifying the comment information based on a text classification technology to determine a category to which the comment information belongs; and generating the cleaned prescription comment information based on the population tag, the diagnosis name, the drug standard information, and the category to which the comment information belongs. A specific example is as shown in Fig. 4a.

For further description, the population tag may include: newborns, children, adults, the elderly, and the like. Common diagnosis name standardization methods include: standardization using an ICD (International Classification of Diseases) 10 code table, training a word vector, and mapping the diagnosis name to a standard disease name in the ICD10 code table using cosine similarity; and extracting a hyponymy of phrases in the diagnosis name using a syntactic analysis algorithm, and using a basic diagnosis name as a standardized disease name of the diagnosis. The category to which the comment information belongs may include: reasonable prescription, inconsistency between diagnosis and medication, inconsistency between medication and gender, repeated medication, very high single dose, very high administration frequency, incorrect administration way, and the like. The performing standardization and unit normalization on the drug information specifically may include: extracting usage and dosage information of a drug from the prescription, standardizing the description of the administration way and the administration frequency in the usage and dosage, and normalizing a unit of the single dose in the usage and dosage. The description of each feature in the prescription comment information is unified, thereby reducing the complexity of the prescription auditing rule.

Step 303: performing feature extraction on prescription information and comment information, to generate a feature data set corresponding to the prescription comment information.

Step 304: encoding the feature data set to obtain a processed feature data set.

In the present embodiment, the executing body may classify and encode relevant features of the diagnosis description in the feature data set generated in step 303, and perform dimension reduction on the code with reference to the package insert to obtain the processed feature data set. Due to the complexity and diversity of the description of the diagnosis name, if ONE-HOT is directly used, very high feature dimension will be caused, thereby failing to contribute to the training of a classification decision model. The feature data set is classified and encoded, to reduce the feature dimension, and solve the problem of sparse diagnosis names.

For example, taking the package insert of finasteride tablet as an example, indications of hair loss and prostate hyperplasia are mentioned in the package insert. Diagnostic information in the prescription involving the finasteride tablet is encoded into a 2-digit code, and the diagnosis name in the prescription is mapped to the 2-digit code using text similarity, so as to solve the problem of sparse diagnosis name. For example, baldness is mapped to hair loss, and prostatitis hyperplasia is mapped to prostate hyperplasia.

Step 305: screening the feature data set based on a preset pharmacological knowledge, to generate a screened feature data set.

In the present embodiment, the executing body may sort and screen the importance of feature dimensions of the feature data set obtained in step 304 based on the preset pharmacological knowledge and in combination with the package insert, to generate the screened feature data set. For example, when a rule of inconsistency between diagnosis and medication is generated, it is only necessary to use a generic name feature of a drug and a diagnostic feature of a patient; when a rule of inconsistency between medication and gender is generated, it is only necessary to use a generic name feature of a drug and a gender feature of a patient; when a rule of repeated medication is generated, it is only necessary to use a generic name feature of a drug and an administration way feature of a drug; when rules, such as very high single dose and very high administration frequency, are generated, it is only necessary to use age, population, and diagnostic features of a patient, and generic name, single dose, and administration frequency features of a drug. Accurate and targeted feature data is obtained by screening, thereby improving the system efficiency and accuracy.

Step 306: judging the feature data set based on a current auditing rule, to obtain a comment result corresponding to the prescription comment information.

In the present embodiment, the executing body may judge the feature data set based on the current comment rule, to obtain the comment result corresponding to the prescription comment information. The auditing rule is updated based on a training result of the classification decision model obtained by training. An example of the auditing rule is as shown in Fig. 4b.

In some alternative implementations of the present embodiment, the method further includes: training the classification decision model based on a preset condition; and the classification decision model is obtained by training based on following steps: acquiring a training sample set, where training samples in the training sample set include: various feature data sets extracted from different hospitals and comment results corresponding to the various feature data sets of different hospitals; and training to obtain the classification decision model using a machine learning method, using the various feature data sets of different hospitals included in the training samples in the training sample set as an input of a detection network, and using the comment results corresponding to the various feature data sets of different hospitals as a desired output of the detection network. The training based on the various feature data sets of different hospitals solves the problem of failure to meet the diverse prescription auditing requirements in a plurality of hospitals because of different auditing rules for the same drug in each hospital, thereby improving the system efficiency and application scope, and improving the system performance using the very strong interpretability of a conventional machine learning model.

In some alternative implementations of the present embodiment, the auditing rule is updated based on the training result of the classification decision model obtained by training, including: judging whether the training of the classification decision model is completed; and updating the auditing rule based on an input parameter and an output parameter of the classification decision model, in response to the training of the classification decision model being completed. The auditing rule is updated after model training is completed each time, such that the auditing rule is consistent with the classification decision model, thereby simplifying the updating process of the auditing rule, whilst guaranteeing the optimal solution of the auditing rule.

In some alternative implementations of the present embodiment, the method further includes: optimizing a product structure and/or a product strategy based on a relevance between the comment result and a product. Based on the comment result, a pharmacist will review the classification decision model and the auditing rule to guarantee the correctness of the auditing result provided by the auditing system.

Specific operations in steps 301, 303, and 306 in the present embodiment are substantially identical to the operations in steps 101, 102, and 103 in the embodiment shown in Fig. 1. The description will not be repeated here.

As can be seen from Fig. 3, compared with the corresponding embodiment of Fig. 1, the schematic diagram 300 of the method for judging information in the present embodiment includes cleaning prescription information and comment information to generate cleaned prescription comment information, encoding a feature data set to obtain a processed feature data set, and screening the feature data set based on a preset pharmacological knowledge to generate a screened feature data set, thereby solving the problem of sparse diagnosis names, reducing the feature dimension, obtaining accurate and targeted feature data by screening, and improving the system efficiency and accuracy.

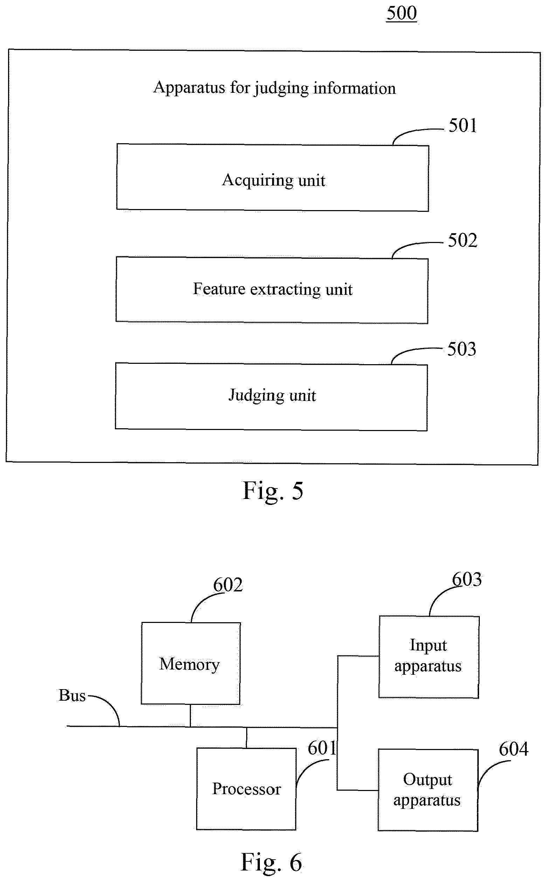

Further referring to Fig. 5, as an implementation of the method shown in the above figures, an embodiment of the present disclosure provides an apparatus for judging information. The embodiment of the apparatus corresponds to the embodiment of the method shown in Fig. 1. The apparatus may be specifically applied to various electronic devices.

As shown in Fig. 5, the apparatus 500 for judging information in the present embodiment includes: an acquiring unit 501, a feature extracting unit 502, and a judging unit 503, where the acquiring unit is configured to acquire prescription comment information, where the prescription comment information includes: prescription information issued by a doctor and comment information given by a pharmacist based on the prescription information; the feature extracting unit is configured to perform feature extraction on the prescription information and the comment information, to generate a feature data set corresponding to the prescription comment information; and the judging unit is configured to judge the feature data set based on a current auditing rule to obtain a comment result corresponding to the prescription comment information, where the auditing rule is used for characterizing a corresponding relationship between the feature data set and the comment result, and the auditing rule is updated based on a training result of a classification decision model obtained by training.

The related description of step 101 to step 103 in the corresponding embodiment of Fig. 1 may be referred to for specific processing of the acquiring unit 501, the feature extracting unit 502, and the judging unit 503 of the apparatus 500 for judging information in the present embodiment and the technical effects thereof, respectively. The description will not be repeated here.

In some alternative implementations of the present embodiment, a filtering unit includes: a clustering module configured to compute, after clustering all product pairs in a first product pair set, a repurchase period of each product in the first product pair set; a first judging module configured to judge whether each product in the first product pair set exceeds a repurchase time limit based on the repurchase period of each product and a purchase moment of the corresponding product; and a first storage module configured to store, if a product in the first product pair set exceeds a repurchase time limit, a product pair of the product in a filtered first product pair set, and store, if the product in the first product pair set does not exceed the repurchase time limit, the product pair of the product in an unfiltered first product pair set.

In some alternative implementations of the present embodiment, the filtering unit further includes: a second judging module configured to judge whether each product in the first product pair set meets an exemption condition based on a moment of occurrence of a user behavior and/or the number of occurrences of the user behavior, where the exemption condition is used for characterizing threshold determination of the moment of occurrence of the user behavior and/or the number of occurrences of the user behavior, and the determination is completed based on a standard product unit (SPU) of the product; and a second storage module configured to store, if the product in the first product pair set meets the exemption condition, the product pair corresponding to the product in the filtered first product pair set, and store, if the product in the first product pair set does not meet the exemption condition, the product pair corresponding to the product in the unfiltered first product pair set.

In some alternative implementations of the present embodiment, the filtering unit further includes: an acquiring module configured to acquire a third product pair set from the first product pair set, where the third product pair set is used for characterizing a set of product pairs without obtaining a repurchase period; and a filtering module configured to filter the third product pair set based on a product repurchase selecting model, to generate a repurchase period of each product in the third product pair set, where the product repurchase selecting model is used for characterizing the selection of the third product pair set based on the product repurchase period.

In some alternative implementations of the present embodiment, a product filtering strategy in a first selecting unit performs combined screening on the unfiltered first product pair set based on a plurality of dimensions; where the first selecting unit includes: a judging module configured to judge whether each product in the unfiltered first product pair set is a purchased commodity of a user based on a product filtering model, where the product filtering model is used for characterizing at least two of a product-based product word, the standard product unit (SPU) of the product, and a stock keeping unit (SKU) of the product, and perform combined filtering on each product in the unfiltered first product pair set; and a deleting module configured to delete, if the product in the unfiltered first product pair set is the purchased product of the user, the product pair corresponding to the product from the unfiltered first product pair set to obtain a second product pair set.

In some alternative implementations of the present embodiment, the apparatus further includes: a second selecting unit configured to select the second product pair set based on a category of the product to obtain a selected second product pair set.

In some alternative implementations of the present embodiment, the apparatus further includes: a generating unit configured to sort a second commodity set of the user based on a commodity display strategy, to generate a commodity list of the user.

According to an embodiment of the present disclosure, the present disclosure further provides an electronic device and a readable storage medium.

As shown in Fig. 6, which is a block diagram of an electronic device of a method for judging information according to an embodiment of the present disclosure. The electronic device is intended to represent various forms of digital computers, such as laptop computers, desktop computers, workbenches, personal digital assistants, servers, blade servers, mainframe computers, and other suitable computers. The electronic device may also represent various forms of mobile apparatuses, such as personal digital processing, cellular phones, smart phones, wearable devices, and other similar computing apparatuses. The components shown herein, their connections and relationships, and their functions are merely examples, and are not intended to limit the implementation of the present disclosure described and/or claimed herein.

As shown in Fig. 6, the electronic device includes: one or more processors 601, a memory 602, and interfaces for connecting various components, including high-speed interfaces and low-speed interfaces. The various components are connected to each other using different buses, and may be installed on a common motherboard or in other methods as needed. The processor may process instructions executed within the electronic device, including instructions stored in or on the memory to display graphic information of GUI (Graphical User Interface) on an external input/output apparatus (such as a display device coupled to the interface). In other embodiments, a plurality of processors and/or a plurality of buses may be used together with a plurality of memories if desired. Similarly, a plurality of electronic devices may be connected, and the devices provide some necessary operations (for example, as a server array, a set of blade servers, or a multi-processor system). In Fig. 6, one processor 601 is used as an example.

The memory 602 is a non-transitory computer readable storage medium provided by the present disclosure. The memory stores instructions executable by at least one processor, so that the at least one processor performs the method for judging information provided by the present disclosure. The non-transitory computer readable storage medium of the present disclosure stores computer instructions for causing a computer to perform the method for judging information provided by the present disclosure.

The memory 602, as a non-transitory computer readable storage medium, may be used to store non-transitory software programs, non-transitory computer executable programs and modules, such as program instructions/modules corresponding to the method for judging information in the embodiments of the present disclosure (for example, the acquiring unit 501, the feature extracting unit 502, and the judging unit 503 shown in Fig. 5). The processor 601 executes the non-transitory software programs, instructions, and modules stored in the memory 602 to execute various functional applications and data processing of the server, that is, to implement the method for judging information in the foregoing method embodiment.

The memory 602 may include a storage program area and a storage data area, where the storage program area may store an operating system and at least one function required application program; and the storage data area may store data created by the use of the electronic device according to the method for judging information, etc. In addition, the memory 602 may include a high-speed random access memory, and may also include a non-transitory memory, such as at least one magnetic disk storage device, a flash memory device, or other non-transitory solid-state storage devices. In some embodiments, the memory 602 may optionally include memories remotely provided with respect to the processor 601, and these remote memories may be connected to the electronic device of the method for judging information through a network. Examples of the above network include but are not limited to the Internet, intranet, local area network, mobile communication network, and combinations thereof.

The electronic device of the method for judging information may further include: an input apparatus 603 and an output apparatus 604. The processor 601, the memory 602, the input apparatus 603, and the output apparatus 604 may be connected through a bus or in other methods. In Fig. 6, connection through a bus is used as an example.

The input apparatus 603 may receive input digital or character information, and generate key signal inputs related to user settings and function control of the electronic device of the method for judging information, such as touch screen, keypad, mouse, trackpad, touchpad, pointing stick, one or more mouse buttons, trackball, joystick and other input apparatuses. The output apparatus 604 may include a display device, an auxiliary lighting apparatus (for example, LED), a tactile feedback apparatus (for example, a vibration motor), and the like. The display device may include, but is not limited to, a liquid crystal display (LCD), a light emitting diode (LED) display, and a plasma display. In some embodiments, the display device may be a touch screen.

Various embodiments of the systems and technologies described herein may be implemented in digital electronic circuit systems, integrated circuit systems, dedicated ASICs (application specific integrated circuits), computer hardware, firmware, software, and/or combinations thereof. These various embodiments may include: being implemented in one or more computer programs that can be executed and/or interpreted on a programmable system that includes at least one programmable processor. The programmable processor may be a dedicated or general-purpose programmable processor, and may receive data and instructions from a storage system, at least one input apparatus, and at least one output apparatus, and transmit the data and instructions to the storage system, the at least one input apparatus, and the at least one output apparatus.

These computing programs (also referred to as programs, software, software applications, or codes) include machine instructions of the programmable processor and may use high-level processes and/or object-oriented programming languages, and/or assembly/machine languages to implement these computing programs. As used herein, the terms "machine readable medium" and "computer readable medium" refer to any computer program product, device, and/or apparatus (for example, magnetic disk, optical disk, memory, programmable logic apparatus (PLD)) used to provide machine instructions and/or data to the programmable processor, including machine readable medium that receives machine instructions as machine readable signals. The term "machine readable signal" refers to any signal used to provide machine instructions and/or data to the programmable processor.

In order to provide interaction with a user, the systems and technologies described herein may be implemented on a computer, the computer has: a display apparatus for displaying information to the user (for example, CRT (cathode ray tube) or LCD (liquid crystal display) monitor); and a keyboard and a pointing apparatus (for example, mouse or trackball), and the user may use the keyboard and the pointing apparatus to provide input to the computer. Other types of apparatuses may also be used to provide interaction with the user; for example, feedback provided to the user may be any form of sensory feedback (for example, visual feedback, auditory feedback, or tactile feedback); and any form (including acoustic input, voice input, or tactile input) may be used to receive input from the user.

The systems and technologies described herein may be implemented in a computing system that includes backend components (e.g., as a data server), or a computing system that includes middleware components (e.g., application server), or a computing system that includes frontend components (for example, a user computer having a graphical user interface or a web browser, through which the user may interact with the implementations of the systems and the technologies described herein), or a computing system that includes any combination of such backend components, middleware components, or frontend components. The components of the system may be interconnected by any form or medium of digital data communication (e.g., communication network). Examples of the communication network include: local area networks (LAN), wide area networks (WAN) and the Internet.

The computer system may include a client and a server. The client and the server are generally far from each other and usually interact through the communication network. The relationship between the client and the server is generated by computer programs that run on the corresponding computer and have a client-server relationship with each other.

The technical solutions according to embodiments of the present disclosure acquire prescription comment information, perform feature extraction on prescription information and comment information to generate a feature data set corresponding to the prescription comment information, and judge the feature data set based on a current auditing rule to obtain a comment result corresponding to the prescription comment information, where the auditing rule is updated based on a training result of a classification decision model obtained by training, thereby solving the problem of incorrect configuration caused by manual rule configuration, freeing the pharmacists from the complex rule configuration work, further solving the problem of failure to dynamically learn the prescription auditing rule because the acquired rule in the prior art is a static rule, implementing a method for judging prescription comment information using an auditing rule obtained by learning, and improving the auditing efficiency and accuracy of the prescription auditing system.

It should be understood that the various forms of processes shown above may be used to reorder, add, or delete steps. For example, the steps described in the present disclosure may be performed in parallel, sequentially, or in different orders. As long as the desired results of the technical solution disclosed in the present disclosure can be achieved, no limitation is made herein.

The above specific embodiments do not constitute limitation on the protection scope of the present disclosure. Those skilled in the art should understand that various modifications, combinations, sub-combinations and substitutions may be made according to design requirements and other factors. Any modification, equivalent replacement and improvement made within the spirit and principle of the present disclosure shall be included in the protection scope of the present disclosure.

## Claims

1. A method for judging information, comprising:
acquiring prescription comment information, wherein the prescription comment information comprises: prescription information issued by a doctor and comment information given by a pharmacist based on the prescription information;
performing feature extraction on the prescription information and the comment information, to generate a feature data set corresponding to the prescription comment information; and
judging the feature data set based on a current auditing rule to obtain a comment result corresponding to the prescription comment information, wherein the auditing rule is used for characterizing a corresponding relationship between the feature data set and the comment result, and the auditing rule is updated based on a training result of a classification decision model obtained by training.

2. The method according to claim 1, wherein the method further comprises:
training the classification decision model based on a preset condition;
and the classification decision model is obtained by training based on following steps:
acquiring a training sample set, wherein training samples in the training sample set comprise: various feature data sets extracted from different hospitals and comment results corresponding to the various feature data sets of different hospitals; and
training to obtain the classification decision model using a machine learning method, using the various feature data sets of different hospitals comprised in the training samples in the training sample set as an input of a detection network, and using the comment results corresponding to the various feature data sets of different hospitals as a desired output of the detection network.

3. The method according to any one of claims 1-2, wherein the auditing rule is updated based on a training result of a classification decision model obtained by training, comprising:
judging whether the training of the classification decision model is completed; and
updating the auditing rule based on an input parameter and an output parameter of the classification decision model, in response to the training of the classification decision model being completed.

4. The method according to any one of claims 1-3, wherein, before the performing feature extraction on the prescription information and the comment information, to generate a feature data set corresponding to the prescription comment information, and before the acquiring a training sample set, the method further comprises:
cleaning the prescription information and the comment information to generate cleaned prescription comment information, wherein the cleaning is based on data structuring on the prescription information and the comment information.

5. The method according to claim 4, wherein the cleaning the prescription information and the comment information to generate cleaned prescription comment information comprises:
performing information extraction on the prescription information to obtain personal information corresponding to the prescription information, diagnostic information corresponding to the prescription information, and drug information corresponding to the prescription information;
determining a population tag corresponding to the prescription information based on the personal information; standardizing the diagnostic information to obtain a diagnosis name corresponding to the prescription information; and performing standardization and unit normalization on the drug information to obtain drug standard information corresponding to the prescription information;
classifying the comment information based on a text classification technology to determine a category to which the comment information belongs; and
generating the cleaned prescription comment information based on the population tag, the diagnosis name, the drug standard information, and the category to which the comment information belongs.

6. The method according to any one of claims 1-5, wherein, after the performing feature extraction on the prescription information and the comment information, to generate a feature data set corresponding to the prescription comment information, and after the acquiring the training sample set, the method further comprises:
encoding the feature data set to obtain a processed feature data set.

7. The method according to any one of claims 1-6, wherein, after the performing feature extraction on the prescription information and the comment information, to generate a feature data set corresponding to the prescription comment information, and after the acquiring the training sample set, the method further comprises:
screening the feature data set based on a preset pharmacological knowledge, to generate a screened feature data set.

8. The method according to any one of claims 1-7, wherein the method further comprises:
optimizing a product structure and/or a product strategy based on a relevance between the comment result and a product.

9. An apparatus for judging information, comprising:
an acquiring unit configured to acquire prescription comment information, wherein the prescription comment information comprises: prescription information issued by a doctor and comment information given by a pharmacist based on the prescription information;
a feature extracting unit configured to perform feature extraction on the prescription information and the comment information, to generate a feature data set corresponding to the prescription comment information; and
a judging unit configured to judge the feature data set based on a current auditing rule to obtain a comment result corresponding to the prescription comment information, wherein the auditing rule is used for characterizing a corresponding relationship between the feature data set and the comment result, and the auditing rule is updated based on a training result of a classification decision model obtained by training.

10. The apparatus according to claim 9, wherein the apparatus further comprises:
a training unit configured to train the classification decision model based on a preset condition; and
the classification decision model in the training unit is obtained by training based on following steps: acquiring a training sample set, wherein training samples in the training sample set comprise: various feature data sets extracted from different hospitals and comment results corresponding to the various feature data sets of different hospitals; and training to obtain the classification decision model using a machine learning method, using the various feature data sets of different hospitals comprised in the training samples in the training sample set as an input of a detection network, and using the comment results corresponding to the various feature data sets of different hospitals as a desired output of the detection network.

11. The apparatus according to any one of claims 9-10, wherein the judging unit comprises:
a judging module configured to judge whether the training of the classification decision model is completed; and
an updating module configured to update the auditing rule based on an input parameter and an output parameter of the classification decision model, in response to the training of the classification decision model being completed.

12. The apparatus according to any one of claims 9-11, wherein the apparatus further comprises:
a cleaning unit configured to clean the prescription information and the comment information to generate cleaned prescription comment information, wherein the cleaning is based on data structuring on the prescription information and the comment information.

13. The apparatus according to claim 12, wherein the cleaning unit comprises:
an extracting module configured to perform information extraction on the prescription information to obtain personal information corresponding to the prescription information, diagnostic information corresponding to the prescription information, and drug information corresponding to the prescription information;
a determining module configured to determine a population tag corresponding to the prescription information based on the personal information; standardizing the diagnostic information to obtain a diagnosis name corresponding to the prescription information; and performing standardization and unit normalization on the drug information to obtain drug standard information corresponding to the prescription information;
a classifying module configured to classify the comment information based on a text classification technology to determine a category to which the comment information belongs; and
a generating module configured to generate the cleaned prescription comment information based on the population tag, the diagnosis name, the drug standard information, and the category to which the comment information belongs.

14. The apparatus according to any one of claims 9-13, wherein the apparatus further comprises:
a processing unit configured to encode the feature data set to obtain a processed feature data set.

15. The apparatus according to any one of claims 9-14, wherein the apparatus further comprises:
a screening unit configured to screen the feature data set based on a preset pharmacological knowledge, to generate a screened feature data set.

16. The apparatus according to any one of claims 9-15, wherein the apparatus further comprises:
an optimizing unit configured to optimize a product structure and/or a product strategy based on a relevance between the comment result and a product.

17. An electronic device, comprising:
at least one processor; and
a memory communicatively connected to the at least one processor; wherein
the memory stores instructions executable by the at least one processor, and the instructions are executed by the at least one processor, such that the at least one processor can execute the method according to any one of claims 1-8.

18. A non-transitory computer readable storage medium storing computer instructions, wherein the computer instructions are used for causing the computer to execute the method according to any one of claims 1-8.
